# FASCICULE DE BREVET EUROPEEN

(11) **EP 4 280 943 B1**
(45) Date de publication et mention de la délivrance du brevet: **23.04.2025**
(21) Numéro de dépôt: 22700593.1
(22) Date de dépôt: 11.01.2022
(51) Int. Cl.: A61B 5/02, A61B 5/0215, A61B 5/00, A61N 1/05

(54) **ENSEMBLE DE DIAGNOSTIC CORONAIRE AVEC GUIDE-FIL SERVANT A LA FOIS POUR L'ASSISTANCE DE STIMULATION CARDIAQUE ET LA MESURE DE LA RESERVE CORONAIRE**
KORONARDIAGNOSTIKEINRICHTUNG MIT DRAHTFÜHRUNG ZUR UNTERSTÜTZUNG DER HERZSTIMULATION UND MESSUNG DER KORONARFLUSSRESERVE
CORONARY DIAGNOSTIC ASSEMBLY WITH WIRE GUIDE FOR BOTH ASSISTING CARDIAC PACING AND MEASURING CORONARY FLOW RESERVE

(30) Priorité: 19.01.2021 FR 2100508
(43) Date de publication de la demande: 29.11.2023
(73) Titulaire: Electroducer, 38000 Grenoble (FR)
(72) Inventeur: FAURIE, Benjamin, 38000 Grenoble (FR)
(74) Mandataire: Cabinet Nony
(86) Numéro de dépôt international: PCT/EP2022/050369
(87) Numéro de publication internationale: WO 2022/157028

(56) Documents cités:
- WO-A1-2020/016565
- FR-A1- 3 079 404
- US-A1- 2014 142 398

## Description

### Domaine technique

La présente invention concerne un ensemble de diagnostic coronaire, et le cas échéant d'angioplastie coronaire.

L'intervention d'angioplastie coronaire est également appelée dilatation transluminale, angioplastie coronaire transluminale percutanée (acronyme anglais PTCA pour « Percutaneous Transluminal Coronary Angioplasty ») ou encore intervention coronarienne percutanée (acronyme anglais PCI pour « Percutaneous Coronary Intervention »).

La présente invention a trait plus particulièrement à l'amélioration du diagnostic d'insuffisance coronaire, notamment en vue d'une angioplastie coronaire.

### Technique antérieure

L'angioplastie coronaire consiste à traiter une artère coronaire rétrécie, par exemple dans le cas de la maladie coronarienne, en la dilatant au moyen d'une sonde munie d'un ballon gonflable à son extrémité.

La sonde montée sur guide métallique est introduite depuis une artère périphérique (radiale ou fémorale) jusque dans l'artère pathologique. Un guide-fil métallique est utilisé pour franchir la sténose ou l'occlusion. Il sert de guide pour positionner de manière stable la sonde d'angioplastie.

Dans la très grande majorité des cas, l'angioplastie au ballonnet est complétée par la mise en place d'un stent, prothèse métallique de faibles dimensions qui est sertie sur le ballonnet.

Avant de réaliser une angioplastie coronaire, le praticien réalise un diagnostic.

Ainsi, dans le cadre de l'étude hémodynamique des sténoses coronariennes, l'utilisation de la physiologie coronaire est très fréquemment nécessaire et a montré son utilité diagnostique et pronostique. Son utilisation largement établie à travers le monde permet d'améliorer le pronostic des patients.

Plus particulièrement, la mesure de la réserve coronaire ou de débit fractionnaire (acronyme anglais FFR pour "Fractional Flow Reserve") est une méthode simple, reconnue, pour évaluer de manière invasive le retentissement fonctionnel d'une lésion coronaire. Le principe de la FFR est basé sur une mesure de pression transsténotique pendant une vasodilatation maximale (hyperhémie), permettant ainsi de quantifier l'impact d'une lésion coronaire spécifique. La mesure FFR est devenue l'indice standard de référence de signification hémodynamique pour guider la revascularisation des lésions coronariennes intermédiaires.

La FFR est facilement mesurée pendant la coronarographie en utilisant un guide de pression pour calculer le rapport de la pression coronarienne distale à une sténose ou un segment malade à la pression aortique dans des conditions d'hyperémie myocardique maximale.

Une FFR de 1.0 est largement acceptée comme normale, Une FFR inférieure à 0,80 est généralement considérée comme associée à une ischémie coronarienne et en faveur d'une revascularisation plutôt que d'une prise en charge conservatrice.

Un FFR peut confirmer ou infirmer une sténose (rétrecissement) coronaire. Une sténose à 70% peut ne pas avoir besoin de stent et inversement une sténose à 50% peut en nécessiter 1.

La méthode standard actuelle de mesure de la FFR consiste à insérer un guide de pression dans l'artère coronaire, tandis que l'agent hyperhémique, généralement l'adénosine, est administré par bolus intracoronarien.

A ce jour, la mise en oeuvre d'une physiologie coronaire, notamment d'une FFR, reste délicate et nécessite comme expliqué ci-avant, l'administration de drogues pour provoquer une hyperhémie, dont la plus connue est l'adénosine. Délivrée par voie intra-coronaire, cette drogue est vectrice d'effets indésirables et secondaires dont les plus fréquents sont des troubles du rythme cardiaque comme des bradycardies ou pauses cardiaques désagréables voir dangereuses générant des malaises de type malaise vagal ou des arrêts cardiaques transitoires chez le patient.

L'utilisation de cette technique est donc parfois limitée par la peur de faire face à ce type de complications ou d'effets secondaires.

Il existe donc un besoin pour améliorer le diagnostic coronaire, plus particulièrement de la physiologie coronaire par FFR, notamment en vue d'une angioplastie, afin de pallier les inconvénients précédents.

Le but de l'invention est de répondre au moins en partie à ce besoin. L'art antérieur pertinent est divulgué dans FR 3 079 404 A1, WO 2020/016565 A1, US 2014/0142398 A1.

### Exposé de l'invention

Pour ce faire, l'invention a pour objet, selon une première alternative, un ensemble de diagnostic coronaire, comprenant :
- un cathéter accessoire, destiné à être introduit dans une artère ou une veine périphérique du corps humain;
- un manchon adapté pour être emmanché autour du cathéter accessoire, le manchon étant en matériau électrique conducteur sur au moins une partie de sa périphérie extérieure de sorte que lorsque le cathéter accessoire est introduit dans l'artère ou dans la veine périphérique du corps humain, la périphérie conductrice du manchon est en contact avec le tissu sous-cutané du corps ou avec la paroi de l'artère ou de la veine, le manchon étant en outre relié à une connexion électrique elle-même reliée à une électrode d'un stimulateur cardiaque externe au corps;
- un guide-fil destiné à être introduit dans la gaine d'introduction du cathéter accessoire, le guide-fil comprenant, dans sa portion distale, un capteur de pression pour la mesure de réserve coronaire et, dans sa portion proximale, une partie métallique servant en outre de connexion à l'autre électrode du stimulateur cardiaque externe ainsi que de connexion au récepteur de pression mesurée par le capteur.

Selon une caractéristique avantageuse, l'électrode du stimulateur cardiaque connectée au manchon conducteur électrique emmanché autour de la gaine d'introduction du cathéter accessoire est l'anode tandis que celle connectée à la partie métallique du guide-fil est la cathode.

Selon une variante de réalisation avantageuse, le manchon électriquement conducteur est constitué d'une pièce monobloc en matériau conducteur, tel que du carbone.

De préférence, le manchon est constitué d'une gaine comprenant sur sa périphérie extérieure un revêtement conducteur électrique, tel qu'un revêtement en carbone.

Avantageusement, le manchon est élastique de sorte à pouvoir s'emmancher sur des gaines de cathéters artériels ou veineux périphériques de différents diamètres, typiquement des diamètres externes compris entre 1,6 et 20 mm.

L'invention a également pour objet, selon une deuxième alternative, un ensemble de diagnostic coronaire, comprenant :
- un cathéter accessoire, destiné à être introduit dans une artère ou une veine périphérique du corps humain, le cathéter accessoire comprenant au moins une gaine tubulaire d'introduction et au moins un élément conducteur électrique dont une portion distale est apparente sur une au moins une partie de la périphérie extérieure de la gaine de sorte à être en contact avec le tissu sous-cutané du corps ou avec la paroi de l'artère ou de la veine périphérique et dont une portion proximale, accessible depuis l'extérieur du corps (C), comprend une connexion électrique de sorte à servir de connexion à une électrode d'un stimulateur cardiaque externe au corps;
- un guide-fil destiné à être introduit dans la gaine d'introduction du cathéter accessoire, le guide-fil comprenant, dans sa portion distale, un capteur de pression pour la mesure de réserve coronaire et, dans sa portion proximale, une partie métallique servant en outre de connexion à l'autre électrode du stimulateur cardiaque externe ainsi que de connexion au récepteur de pression mesurée par le capteur..

Le cathéter accessoire peut être un introducteur introduit dans l'artère radiale ou fémorale d'un patient.

Selon une caractéristique avantageuse, l'électrode du stimulateur cardiaque connectée au cathéter accessoire est l'anode tandis que celle connectée à la partie métallique du guide-fil est la cathode.

Selon une variante de réalisation avantageuse, l'élément conducteur électrique du cathéter accessoire est un fil ou une bande métallique logé(e) au moins en partie dans l'épaisseur de la gaine dont une portion distale est apparente à la périphérie extérieure de la gaine.

Avantageusement, la section du fil ou de la bande métallique est comprise entre 0,25 et 5 mm².

Le capteur de pression peut être un capteur piézoélectrique ou à fibre optique, qui est relié à l'unité centrale de mesure de pression respectivement par un fil électrique ou une fibre optique. L'inventeur a vaincu un préjugé sur ce point car, générer un courant électrique pour la stimulation cardiaque recherchée se rajoute au signal électrique ou par fibre optique pour la mesure de pression. Et il s'avère qu'aucune perturbation n'est induite, i.e. la mesure de pression n'est pas perturbée par le signal électrique de la stimulation cardiaque.

L'ensemble selon l'invention peut constituer en outre un ensemble d'angioplastie coronaire avec pose d'un stent, le guide-fil étant adapté pour réaliser l'avancement du stent.

L'invention concerne également une méthode de diagnostic et le cas échéant d'intervention cardiaque, comprenant les étapes suivantes :
i/ emmanchement d'un manchon autour d'un cathéter accessoire, le manchon étant en matériau électrique conducteur sur au moins une partie de sa périphérie extérieure et comprenant en outre une connexion électrique à une électrode d'un stimulateur cardiaque externe au corps ;
ii/ introduction d'un cathéter accessoire dans une artère ou une veine périphérique d'un corps humain, de sorte que la périphérie conductrice du manchon est en contact avec le tissu sous-cutané du corps ou avec la paroi de l'artère ou de la veine;
iii/ introduction d'un guide-fil dans la gaine d'introduction du cathéter accessoire, le guide-fil comprenant à son extrémité distale un capteur de pression et, à sa partie proximale une partie métallique servant en outre de connexion à l'autre électrode du stimulateur cardiaque externe ;
iv/ mesure de la pression à l'aide du capteur en tant que mesure de la réserve coronaire et simultanément à la mesure de la réserve coronaire, stimulation cardiaque directe sur le guide-fil au moyen du stimulateur cardiaque externe;
v/ le cas échéant, en cas de pose de stent, stimulation de cardiaque au moyen du stimulateur cardiaque externe, le guide-fil étant maintenu en place une fois l'étape iv/ effectuée.

L'invention concerne enfin une méthode de diagnostic et le cas échéant d'intervention cardiaque, comprenant les étapes suivantes :
i'/ introduction d'un cathéter accessoire dans une artère ou une veine périphérique d'un corps humain, le cathéter accessoire comprenant au moins une gaine tubulaire d'introduction et au moins un élément conducteur électrique dont une portion distale est apparente sur une au moins une partie de la périphérie extérieure de la gaine de sorte à être en contact avec le tissu sous-cutané du corps ou avec la paroi de l'artère ou de la veine périphérique et dont une portion proximale, accessible depuis l'extérieur du corps (C), comprend une connexion électrique de sorte à servir de connexion à une électrode d'un stimulateur cardiaque externe au corps;
ii'/ introduction d'un guide-fil dans la gaine d'introduction du cathéter accessoire, le guide-fil comprenant, à son extrémité distale, un capteur de pression et, à son extrémité proximale, une partie métallique servant en outre de connexion à l'autre électrode du stimulateur cardiaque externe ;
iii'/ mesure de la pression à l'aide du capteur en tant que mesure de la réserve coronaire et simultanément à la mesure de la réserve coronaire, stimulation cardiaque directe sur le guide-fil au moyen du stimulateur cardiaque externe;
iv'/ le cas échéant, en cas de pose du stent, stimulation de cardiaque au moyen du stimulateur cardiaque externe, le guide-fil étant maintenu en place une fois l'étape iii'/ effectuée.

Ainsi, l'invention consiste essentiellement en un ensemble de diagnostic coronaire dont le guide-fil sert à la fois à mesurer des pressions transsténotiques pour une réserve coronaire (FFR), par le capteur de pression intégré dans le guide-fil et à réaliser la stimulation cardiaque lors de cette mesure FFR.

En induisant une stimulation cardiaque directe sur un guide-fil destiné à une mesure FFR, l'ensemble selon invention permet de se dédouaner d'effets indésirables et secondaires, liés aux troubles du rythme cardiaque ou de conduction, tels qu'une bradycardie ou pause cardiaque délétère, de l'hyperhémie qu'il est nécessaire de réaliser au préalable d'une mesure FFR.

Ainsi, l'invention sécurise la procédure de mesure FFR, le praticien et le patient et permet de réaliser ce type d'étude physiologique chez tout type de patient même en cas de contre-indication théorique à l'utilisation d'adénosine nécessaire pour réaliser une hyperhémie. Un exemple de contre-indication est un bloc auriculo-ventriculaire de haut grade.

En outre, l'intensité de stimulation nécessaire à la sidération cardiaque lors de la mesure FFR est faible. Typiquement, l'intensité du courant délivré, en vue d'une sidération cardiaque, peut aller de 4 à 25 mA et la tension délivrée de 1 à 15 Volt.

De ce fait, on garantit non seulement que le patient est protégé des effets indésirables et secondaires (brachycardie, pause cardiaque), mais en outre le patient ne ressent peu voire aucune sensation liée à la stimulation cardiaque, étant donné que l'électrode endovasculaire, typiquement l'anode est de grandes dimensions.

Le(s) chirurgien(s) en charge de l'opération peu(ven)t ainsi relier l'électrode, typiquement l'anode du stimulateur cardiaque au manchon conducteur emmanché autour du cathéter accessoire facilement puis relier comme usuellement l'autre électrode, typiquement la cathode au guide-fil de l'ensemble cathéter-stent ou ballon seul. Cela est important en particulier pour des patients plus fragiles.

En résumé, les avantages d'un ensemble de diagnostic coronaire sont nombreux parmi lesquels on peut citer:
- une sécurisation de la procédure de mesure coronaire FFR en supprimant tous les effets secondaires et indésirables liés à l'ingestion de drogues telles que l'adénosine nécessaire pour réaliser l'hyperhémie préalable à la FFR;
- une garantie de la stimulation temporaire en vue de réaliser une stimulation « de secours », c'est-à-dire en situation d'urgence, souhaitée du fait de la moindre impédance du système vasculaire rencontré par le courant électrique de stimulation puisque le manchon autour d'un cathéter veineux accessoire ou ce dernier lorsqu'il intégre la partie métallique à relier à l'électrode est directement en contact avec ledit système;
- possibilité de mise en œuvre sur tout cathéter veineux ou artériel périphérique existant, puisque seul un manchon conducteur doit être emmanché autour d'un cathéter veineux ou artériel périphérique, avant son introduction dans une veine ou artère périphérique et une connexion électrique sur le guide-fil insérée dans le cathéter doit être effectuée.

La seule relative contrainte du manchon l'est au début de l'intervention lors de la préparation et consiste en un emmanchement du manchon autour de la gaine d'introduction du cathéter accessoire (artériel périphérique).

Mais cette opération est très simple et aisée à mettre en œuvre et peut tout-à-fait être réalisée par un(e) assistant(e) ou infirmier(ère), celui-ci (celle-ci) n'ayant pas besoin d'avoir de compétences particulières pour cette tâche.

D'autres avantages et caractéristiques ressortiront mieux à la lecture de la description détaillée, faite à titre illustratif et non limitatif, en référence aux figures suivantes.

### Brève description des dessins

[Fig 1] la figure 1 est une vue schématique illustrant l'utilisation d'un ensemble de diagnostic selon l'invention avec un cathéter artériel périphérique et un manchon de stimulation selon l'invention, le cathéter étant introduit directement dans une artère périphérique d'un patient.
[Fig 2] la figure 2 montre l'agencement du guide-fil de l'ensemble selon la figure 1 au sein d'une artère coronaire à diagnostiquer.
[Fig 3] la figure 3 est une vue en perspective d'un manchon électriquement conducteur selon l'invention destiné à être emmanché autour d'un cathéter artériel périphérique comme représente en figures 1 et 2;
[Fig 4] la figure 4 est une vue de coupe longitudinale partielle montrant un manchon selon la figure 3 emmanché autour d'un cathéter artériel périphérique.

### Description détaillée

Dans la description qui va suivre ainsi que dans l'ensemble de la présente demande, les termes « distal » et « proximal » sont utilisés par référence avec le corps d'un patient pour lequel un diagnostic coronaire, le cas échéant suivi d'une angioplastie coronaire. Ainsi, l'extrémité distale d'un guide-fil est l'extrémité située le plus à l'intérieur du patient lors du diagnostic.

On précise que les différents éléments ne sont pas nécessairement représentés à l'échelle.

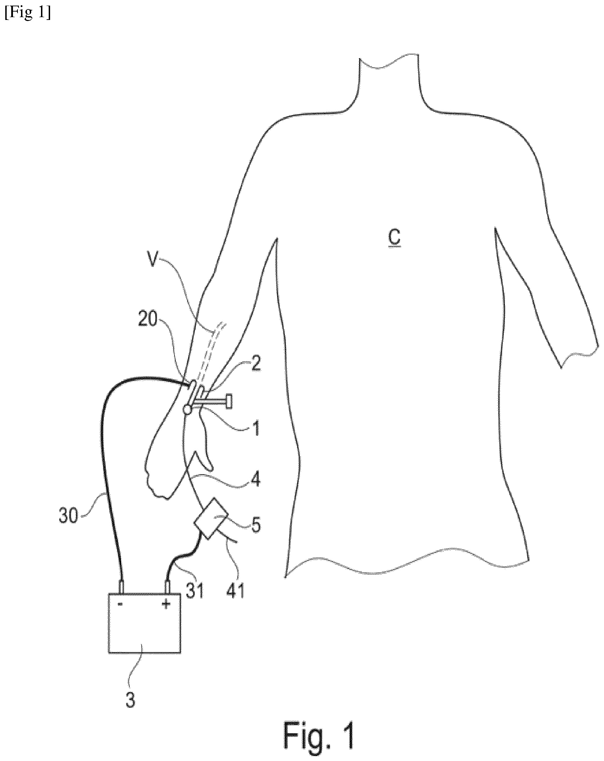

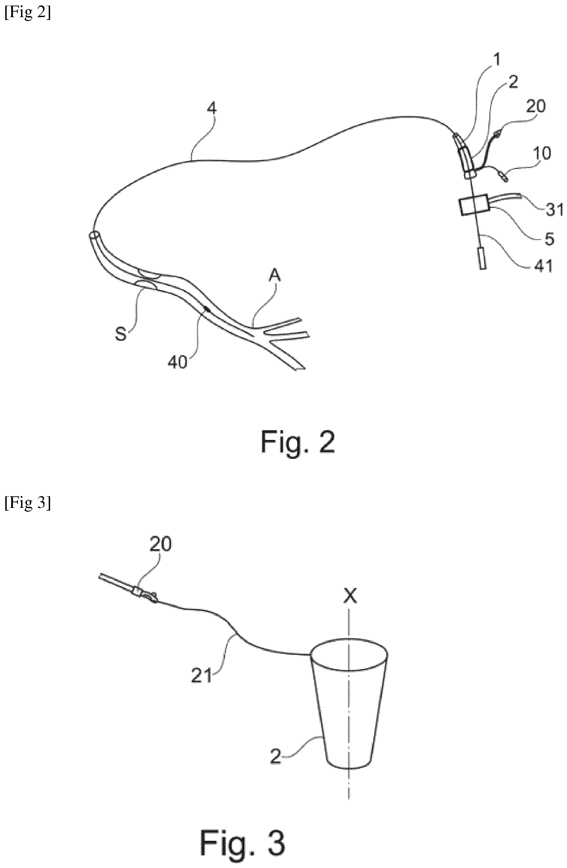

On a représenté en figures 1 et 2 un ensemble de diagnostic coronaire selon l'invention.

Un cathéter artériel périphérique 1 aussi appelé cathéter accessoire est introduit dans une artère périphérique V.

Un tel cathéter artériel 1 qui peut être de faible diamètre, typiquement de 2mm ou moins, et de faible longueur. Il est conforme aux normes relatives aux cathéters intravasculaires périphériques existants. Le cathéter 1 peut comprendre un dispositif de rinçage 10 à robinets, couramment appelé «flush», intégré pour rincer l'intérieur du cathéter 1 au moyen d'un liquide de rinçage approprié.

Un manchon conducteur électriquement 2 est emmanché directement autour du cathéter artériel périphérique 1. Le manchon 2 est relié à une connexion électrique 20 elle-même reliée à une électrode, typiquement l'anode d'un stimulateur cardiaque 3, externe au corps C, par le biais d'un fil d'alimentation électrique 30.

Un guide-fil 4 est introduit dans la gaine d'introduction du cathéter accessoire 1.

Comme montré en figure 2, le guide-fil 4 comprend, dans sa portion distale, un capteur de pression 40 pour la mesure de pressions transsténotique dans l'artère coronaire A et, dans sa portion proximale, une partie métallique 41 servant en outre de connexion à l'autre électrode, typiquement la cathode du stimulateur cardiaque externe. Pour assurer la connexion électrique, une pince 5 de type crocodile est fixée par pincement sur la partie métallique 41 du guide-fil 4. Cette pince 5 est reliée à l'anode du stimulateur cardiaque par le biais d'un fil d'alimentation électrique 31.

Un manchon 2 selon l'invention est représenté en figure 3 : il se présente sous la forme d'un tube dont au moins une partie de sa périphérie extérieure est électriquement conductrice.

Cette partie conductrice est reliée à une connexion électrique 20, typiquement sous la forme d'une pince par le biais d'un fil d'alimentation électrique 21. Le manchon 20, la pince 20 et le fil d'alimentation électrique 21 peuvent avantageusement constituer un kit prêt à être emmanché autour d'un cathéter 1 et connecté à une électrode de stimulateur cardiaque 3.

Le manchon 2 peut être de forme cylindrique ou en tronc de cône. Sa forme vient épouser au plus près celle de la forme extérieure de la gaine d'introduction d'un cathéter artériel périphérique 1.

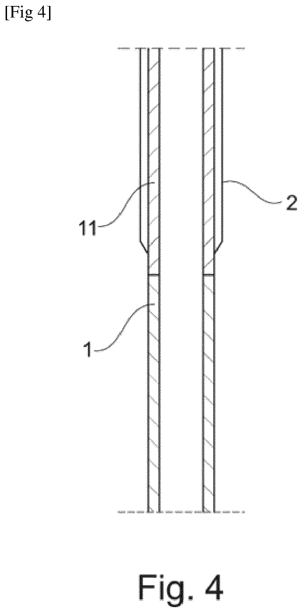

Comme visible sur la figure 4, l'épaisseur de ce manchon 2, typiquement de l'ordre du millimètre ou inférieure, ne rajoute qu'une faible surépaisseur à la gaine 11 du cathéter artériel 1 et donc ne gêne pas la progression celle-ci lors de son introduction dans une artère périphérique V.

Le manchon 2 peut être réalisé sous la forme d'une pièce monobloc entièrement conductrice ou sous la forme d'une pièce revêtue d'un revêtement conducteur électrique. En tant que matériau électriquement conducteur, on peut choisir avantageusement du carbone.

Selon une variante avantageuse, on peut concevoir le manchon 2 afin qu'il soit élastique et qu'il puisse ainsi s'adapter à toute taille de cathéter artériel périphérique existant.

En pratique, un chirurgien ou médecin interventionniste souhaitant procéder à un diagnostic coronaire avec stimulation cardiaque concomitante comme expliqué par la suite, commence par mettre en place le manchon conducteur 2 autour d'un cathéter artériel périphérique 1. La mise en place du manchon conducteur étant très simple et aisée, elle peut être faite par une assistante ou infirmière sans qu'elle n'ait besoin d'adopter une technique particulière.

La mise en place est atteinte une fois que le manchon 2 est emmanché et agencé autour de la gaine d'introduction du cathéter artériel 1 de sorte que ledit manchon 2 vienne toucher soit une zone sous cutanée du patient, soit la paroi la veine périphérique du patient.

Une fois cette mise en place effectuée, la connexion électrique 20 peut être connectée directement à l'anode d'un stimulateur cardiaque externe 3 par le biais d'un fil de connexion 30.

Usuellement, une pince 5, comme une pince-crocodile 3 peut à son tour être est fixée par pincement sur la partie métallique 41 du guide-fil 4 inséré dans la gaine 11 du cathéter périphérique 1 et dont le capteur de pression 40 est positionné dans l'artère coronaire à diagnostiquer. Cette pince 5 est reliée à la cathode du stimulateur cardiaque externe 3 par le biais d'un fil de connexion 31.

Ainsi, la stimulation cardiaque temporaire pour réaliser la sidération cardiaque souhaitée au cours d'une mesure de pression transsténotique, peut avoir lieu entre la cathode reliée électriquement au guide-fil 4 et l'anode reliée électriquement au manchon 2 selon l'invention autour du cathéter veineux périphérique 1.

On décrit maintenant la méthode de diagnostic coronaire et le cas échéant d'intervention cardiaque, mise en oeuvre par l'ensemble décrit précédemment.

Cette méthode est appliquée lorsqu'un praticien souhaite faire un diagnostic coronaire, notamment une mesure de la réserve coronaire parce qu'il suspecte une sténose dans une artère coronaire A, telle que la sténose identifiée S en figure 2.

Etape i/: une infirmière ou un praticien procède à l'emmanchement du manchon électriquement conducteur 2 autour d'un cathéter périphérique (accessoire) 2.

Etape ii/: le praticien réalise alors l'introduction du cathéter 1 dans une artère ou une veine périphérique d'un corps humain C, de sorte que la périphérie conductrice du manchon 2 est en contact avec le tissu sous-cutané du corps ou avec la paroi de l'artère ou de la veine.

Etape iii/: le praticien introduit ensuite le guide-fil 4 dans la gaine d'introduction 11 du cathéter accessoire 1, jusqu'à s'assurer que le capteur de pression 40 est bien positionné dans l'artère coronaire A à diagnostiquer.

Il peut alors procéder à la connexión électrique au stimulateur cardiaque externe 3.

Ainsi, l'infirmière ou la praticien connecte d'une part le manchon 2 à une électrode, typiquement l'anode du stimulateur cardiaque externe 3 et d'autre part la partie métallique 40 du guide-fil 4 à l'autre électrode du stimulateur 3.

Etape iv/: après avoir provoqué une hyperhémie du patient, le praticien peut mesurer la pression transsténotique à l'aide du capteur 40 en tant que mesure de la réserve coronaire.

Simultanément à la mesure de la réserve coronaire FFR, une stimulation cardiaque directe est effectuée sur le guide-fil 4.

Plus précisément, la pression mesurée FFR l'est une fois que le praticien s'est assuré que l'adénosine administré au patient a fait son effet.

La stimulation cardiaque peut être déclenchée avant l'initiation de la mesure de la pression, typiquement avant ou en même temps que le praticien s'est assuré de l'effet de l'adénosine. Le praticien peut aussi décider de faire l'administration d'adénosine sous stimulation cardiaque au moyen du guide-fil selon l'invention.

En ce qui concerne la cadence de stimulation cardiaque, le praticien peut choisir parmi l'un des deux modes suivants:
- un mode de stimulation que l'on peut qualifier de "sentinelle": le stimulateur cardiaque externe est réglé à un seuil de rythme inférieur à celui du coeur du patient au repos. Dès que le battement du coeur du patient devient inférieur au seuil, alors le stimulateur est mis en fonctionnement et la stimulation cardiaque est effective via l'électrode du guide-fil et l'électode intégrée au manchon ou dans le cathéter accessoire. Par exemple, le coeur d'un patient peut battre au repos à 80 pulsations par minute et dès qu'au cours du diagnostic FFR, il bat à 60 alors le stimulateur cardiaque externe est déclenché;
- un mode de stimulation permanente selon lequel le stimulateur cardiaque externe est déclenché à permanence au cours du diagnostic FFR et le cas échéant dès l'administration de l'adénosine, à une fréquence supérieure au rythme cardiaque du patient au repos. Par exemple si le rythme du patient au repos est à 60 pulsations par minute, alors le stimulateur cardiaque externe fonctionne en permanence à un rythme de 80 pulsations par minute.

Quelle que soit le mode choisi par le praticien, cette cadence de stimulation peut être aisément mise en oeuvre sachant que, selon la procédure FFR, le rythme cardiaque du patient est monitoré initialement et en permanence pendant toute l'intervention (diagnostic éventuellement suivi d'une angioplastie coronaire).

En procédant ainsi à une stimulation cardiaque simultanément, on évite pour le patient tous les risques liés à une bradycardie ou une pause cardiaque délétère qui serait provoquée par l'hyperhémie.

L'ensemble de diagnostif coronaire qui vient d'être décrit peut en outre servir à réaliser une angioplastie coronaire.

En effet, à l'issue de l'étape iv/, si une sténose S identifiée nécessite la pose d'un stent dans l'artère coronaire A, alors en ayant choisi un guide-fil 4 qui intègre en outre un ballonnet gonflable avec stent, on peut réaliser sans avoir enlevé aucun des composants de l'ensemble, la mise en place du stent.

Ainsi, selon une étape v/, lors du déploiement du ballonnet et du stent, le praticien procede à une autre stimulation de cardiaque au moyen du stimulateur cardiaque externe, le guide-fil étant maintenu en place une fois l'étape iv/ effectuée.

L'invention n'est pas limitée aux exemples qui viennent d'être décrits ; on peut notamment combiner entre elles des caractéristiques des exemples illustrés au sein de variantes non illustrées.

D'autres variantes et améliorations peuvent être prévues sans pour autant sortir du cadre de l'invention.

Si dans l'ensemble des exemples illustrés, le guide-fil intégrant à la fois un capteur de pression et une partie métallique en tant qu'électrode de stimulation cardiaque externe est mis en œuvre dans un ensemble de diagnostic coronaire, il peut tout-à-fait être mis en œuvre pour une prise de pression transsténotique dans des cavités cardiaques et une stimulation cardiaque comme selon l'enseignement des demandes de brevet FR3079404, EP3522800, EP3280338 (B1) FR3057153, WO2019/185880, dans le cadre d'une implantation d'une valve aortique par voie percutanée (acronyme anglais TAVI pour « Transcatheter Aortic Valve Implantation »).

Un guide-fil pour un ensemble de diagnostic coronaire (FFR) peut avoir un faible diamètre, typiquement égal à 0,035 mm (0,014 inch) tandis qu'un guide-fil pour un ensemble de prise de pression au sein de cavités cardiaques peut être de plus grand diamètre, typiquement de l'ordre de 0,9 mm (0,35 inch).

## Revendications

1. Ensemble de diagnostic coronaire, comprenant :
- un cathéter accessoire (1), destiné à être introduit dans une artère ou une veine périphérique du corps humain;
- un manchon (2) adapté pour être emmanché autour du cathéter accessoire, le manchon étant en matériau électrique conducteur sur au moins une partie de sa périphérie extérieure de sorte que lorsque le cathéter accessoire est introduit dans l'artère ou dans la veine périphérique du corps humain, la périphérie conductrice du manchon est en contact avec le tissu sous-cutané du corps ou avec la paroi de l'artère ou de la veine, le manchon étant en outre relié à une connexion électrique elle-même reliée à une électrode d'un stimulateur cardiaque externe au corps (3);
- un guide-fil (4) destiné à être introduit dans une gaine d'introduction (11) du cathéter accessoire, le guide-fil comprenant, dans sa portion distale, un capteur de pression (40) pour la mesure de réserve coronaire et, dans sa portion proximale, une partie métallique (41) servant en outre de connexion à l'autre électrode du stimulateur cardiaque externe ainsi que de connexion au récepteur de pression mesurée par le capteur.

2. Ensemble selon la revendication 1, l'électrode du stimulateur cardiaque connectée au manchon conducteur électrique emmanché autour de la gaine d'introduction du cathéter accessoire étant l'anode tandis que celle connectée à la partie métallique du guide-fil est la cathode.

3. Ensemble selon l'une des revendications 1 ou 2, dans lequel le manchon électriquement conducteur est constitué d'une pièce monobloc en matériau conducteur, tel que du carbone.

4. Ensemble selon revendication 3, le manchon étant constitué d'une gaine comprenant sur sa périphérie extérieure un revêtement conducteur électrique, tel qu'un revêtement en carbone.

5. Ensemble selon la revendication 3 ou 4, le manchon étant élastique de sorte à pouvoir s'emmancher sur des gaines de cathéters artériels ou veineux périphériques de différents diamètres, typiquement des diamètres externes compris entre 1,6 et 20 mm.

6. Ensemble de diagnostic coronaire, comprenant :
- un cathéter accessoire, destiné à être introduit dans une artère ou une veine périphérique du corps humain, le cathéter accessoire comprenant au moins une gaine tubulaire d'introduction et au moins un élément conducteur électrique dont une portion distale est apparente sur une au moins une partie de la périphérie extérieure de la gaine de sorte à être en contact avec le tissu sous-cutané du corps ou avec la paroi de l'artère ou de la veine périphérique et dont une portion proximale, accessible depuis l'extérieur du corps (C), comprend une connexion électrique de sorte à servir de connexion à une électrode d'un stimulateur cardiaque externe au corps;
- un guide-fil destiné à être introduit dans la gaine d'introduction du cathéter accessoire, le guide-fil comprenant, dans sa portion distale, un capteur de pression pour la mesure de réserve coronaire et, dans sa portion proximale, une partie métallique servant en outre de connexion à l'autre électrode du stimulateur cardiaque externe ainsi que de connexion au récepteur de pression mesurée par le capteur.

7. Ensemble selon la revendication 6, l'électrode du stimulateur cardiaque connectée au cathéter accessoire étant l'anode tandis que celle connectée à la partie métallique du guide-fil est la cathode.

8. Ensemble selon l'une des revendications 6 ou 7, dans lequel l'élément conducteur électrique du cathéter accessoire est un fil ou une bande métallique logé(e) au moins en partie dans l'épaisseur de la gaine dont une portion distale est apparente à la périphérie extérieure de la gaine.

9. Ensemble selon la revendication 8, la section du fil ou de la bande métallique étant comprise entre 0,25 et 5 mm².

10. Ensemble selon l'une des revendications précédentes, le capteur de pression étant un capteur piézoélectrique ou à fibre optique.

11. Ensemble selon l'une des revendications précédentes, constituant en outre un ensemble d'angioplastie coronaire avec pose d'un stent, le guide-fil étant adapté pour réaliser l'avancement du stent.

## Patentansprüche

1. Anordnung zur Koronardiagnostik, umfassend:
- einen Zubehörkatheter (1), der dazu bestimmt ist, in eine Arterie oder eine periphere Vene des menschlichen Körpers eingeführt zu werden;
- eine Hülse (2), die geeignet ist, um den Zubehörkatheter herum aufgeschoben zu werden, wobei die Hülse über mindestens einen Teil ihres Außenumfangs aus elektrisch leitendem Material ist, so dass, wenn der Zubehörkatheter in die Arterie oder in die periphere Vene des menschlichen Körpers eingeführt ist, der leitende Umfang der Hülse mit dem subkutanen Gewebe des Körpers oder mit der Wand der Arterie oder der Vene in Kontakt ist, wobei die Hülse ferner mit einem elektrischen Anschluss verbunden ist, der wiederum mit einer Elektrode eines in Bezug auf den Körper externen Herzstimulators (3) verbunden ist;
- einen Führungsdraht (4), der dazu bestimmt ist, in eine Einführhülle (11) des Zubehörkatheters eingeführt zu werden, der Führungsdraht umfassend, in seinem distalen Abschnitt, einen Drucksensor (40) zum Messen der Koronarreserve und, in seinem proximalen Abschnitt, einen metallischen Teil (41), der ferner als Anschluss an die andere Elektrode des externen Herzstimulators sowie als Anschluss an den Empfänger für den von dem Sensor gemessenen Druck dient.

2. Anordnung nach Anspruch 1, wobei die Elektrode des Herzstimulators, die an die um die Einführhülle des Zubehörkatheters aufgeschobene elektrisch leitende Hülse angeschlossen ist, die Anode ist, während die an den metallischen Teil des Führungsdrahts angeschlossene die Kathode ist.

3. Anordnung nach einem der Ansprüche 1 oder 2, wobei die elektrisch leitende Hülse aus einem einstückigen Teil aus leitendem Material wie etwa Kohlenstoff besteht.

4. Anordnung nach Anspruch 3, wobei die Hülse aus einer Hülle besteht, die an ihrem Außenumfang einen elektrisch leitenden Überzug wie einen Kohlenstoffüberzug umfasst.

5. Anordnung nach Anspruch 3 oder 4, wobei die Hülse elastisch ist, so dass sie auf Hüllen von peripheren Arterien- oder Venenkathetern mit unterschiedlichen Durchmessern, typischerweise Außendurchmessern zwischen 1,6 und 20 mm, aufgeschoben werden kann.

6. Anordnung zur Koronardiagnostik, umfassend:
- einen Zubehörkatheter, der dazu bestimmt ist, in eine Arterie oder eine periphere Vene des menschlichen Körpers eingeführt zu werden, der Zubehörkatheter umfassend mindestens eine röhrenförmige Einführhülle und mindestens ein elektrisch leitendes Element, von dem ein distaler Abschnitt über mindestens einen Teil des Außenumfangs der Hülle frei liegt, so dass er mit dem subkutanen Gewebe des Körpers oder mit der Wand der Arterie oder der peripheren Vene in Kontakt ist, und von dem ein proximaler Abschnitt, der von außerhalb des Körpers (C) zugänglich ist, einen elektrischen Anschluss umfasst, so dass er als Anschluss an eine Elektrode eines in Bezug auf Körper externen Herzstimulators dienen kann;
- einen Führungsdraht, der dazu bestimmt ist, in die Einführhülle des Zubehörkatheters eingeführt zu werden, der Führungsdraht umfassend, in seinem distalen Abschnitt, einen Drucksensor zum Messen der Koronarreserve und, in seinem proximalen Abschnitt, einen metallischen Teil, der ferner als Anschluss an die andere Elektrode des externen Herzstimulators sowie als Anschluss an den Empfänger des von dem Sensor gemessenen Drucks dient.

7. Anordnung nach Anspruch 6, wobei die an den Zubehörkatheter angeschlossene Elektrode des Herzstimulators die Anode ist, während die an den metallischen Teil des Führungsdrahts angeschlossene die Kathode ist.

8. Anordnung nach einem der Ansprüche 6 oder 7, wobei das elektrisch leitende Element des Zubehörkatheters ein mindestens zum Teil in der Dicke der Hülle aufgenommener Draht oder Metallstreifen ist, von dem ein distaler Abschnitt am Außenumfang der Hülle frei liegt.

9. Anordnung nach Anspruch 8, wobei der Querschnitt des Drahts oder Metallstreifens zwischen 0,25 und 5 mm² beträgt.

10. Anordnung nach einem der vorhergehenden Ansprüche, wobei der Drucksensor ein piezoelektrischer Sensor oder ein Sensor mit optischer Faser ist.

11. Anordnung nach einem der vorhergehenden Ansprüche, die ferner eine Anordnung zur Koronarangioplastie mit Setzen eines Stents bildet, wobei der Führungsdraht geeignet ist, den Vorschub des Stents auszuführen.

## Claims

1. Coronary diagnostic assembly, comprising:
- an accessory catheter (1), intended to be introduced into a peripheral artery or vein of the human body;
- a sleeve (2) adapted to be engaged around the accessory catheter, the sleeve being made of electrically conductive material over at least part of its outer periphery, such that, when the accessory catheter is introduced into the peripheral artery or vein of the human body, the conductive periphery of the sleeve is in contact with the subcutaneous tissue of the body or with the wall of the artery or of the vein, the sleeve being additionally connected to an electrical connection, itself connected to an electrode of a cardiac stimulator (3) outside the body;
- a guidewire (4) intended to be introduced into an insertion sheath (11) of the accessory catheter, the guidewire comprising, in its distal portion, a pressure sensor (40) for measuring coronary reserve and, in its proximal portion, a metal part (41) further serving as a connection to the other electrode of the external cardiac stimulator and also as a connection to the receiver of the pressure measured by the sensor.

2. Assembly according to Claim 1, the electrode of the cardiac stimulator connected to the electrically conductive sleeve engaged around the insertion sheath of the accessory catheter being the anode, while the one connected to the metal part of the guidewire is the cathode.

3. Assembly according to either of Claims 1 and 2, in which the electrically conductive sleeve is formed as a single piece made of conductive material, for example carbon.

4. Assembly according to Claim 3, the sleeve being formed by a sheath comprising on its outer periphery an electrically conductive coating, for example a coating of carbon.

5. Assembly according to Claim 3 or 4, the sleeve being elastic so as to be able to engage on peripheral arterial or venous catheter sheaths of different diameters, typically external diameters of between 1.6 and 20 mm.

6. Coronary diagnostic assembly, comprising:
- an accessory catheter, intended to be introduced into a peripheral artery or vein of the human body, the accessory catheter comprising at least one tubular insertion sheath and at least one electrically conductive element, of which a distal portion is exposed on at least one part of the outer periphery of the sheath in such a way as to be in contact with the subcutaneous tissue of the body or with the wall of the peripheral artery or vein, and of which a proximal portion, accessible from outside the body (C), comprises an electrical connection so as to serve as a connection to an electrode of a cardiac stimulator outside the body;
- a guidewire intended to be introduced into the insertion sheath of the accessory catheter, the guidewire comprising, in its distal portion, a pressure sensor for measuring the coronary reserve and, in its proximal portion, a metal part further serving as a connection to the other electrode of the external cardiac stimulator and also as a connection to the receiver of the pressure measured by the sensor.

7. Assembly according to Claim 6, the electrode of the cardiac stimulator connected to the accessory catheter being the anode, while the one connected to the metal part of the guidewire is the cathode.

8. Assembly according to either of Claims 6 and 7, in which the electrically conductive element of the accessory catheter is a wire or a metal band housed at least partially within the thickness of the sheath, of which a distal portion is exposed at the outer periphery of the sheath.

9. Assembly according to Claim 8, the cross section of the wire or of the metal band being between 0.25 and 5 mm².

10. Assembly according to one of the preceding claims, the pressure sensor being a piezoelectric or fibre-optic sensor.

11. Assembly according to one of the preceding claims, additionally constituting a coronary angioplasty assembly with placement of a stent, the guidewire being adapted for advancing the stent.
